(19) Europäisches Patentamt
European Patent Office
Office européen des brevets

(11) Publication number: **0 510 197 A1**

(12) **EUROPEAN PATENT APPLICATION published in accordance with Art. 158(3) EPC**

(21) Application number: **91901901.8**

(22) Date of filing: **09.01.91**

(86) International application number: **PCT/JP91/00006**

(87) International publication number: **WO 91/10431 (25.07.91 91/17)**

(51) Int. Cl.[5]: **A61K 31/40**, A61K 9/107, A61K 9/127, A61K 9/14

(30) Priority: **11.01.90 JP 5203/90**

(43) Date of publication of application: **28.10.92 Bulletin 92/44**

(84) Designated Contracting States: **AT BE CH DE DK ES FR GB GR IT LI LU NL SE**

(71) Applicant: **NIPPON SHINYAKU COMPANY, LIMITED**
**14, Kisshoin Nishinosho Monguchicho**
**Minami-ku Kyoto-shi Kyoto 601(JP)**

(72) Inventor: **SEKI, Junzo**
**6-16, Shirogane 3-chome Inagawacho**
**Kawabe-gun Hyogo 666-02(JP)**
Inventor: **USHIMARU, Kouichi**
**17, Kamitachiuridori Muromachinishiiru**
**Kamikyo-ku**
**Kyoto-shi Kyoto 602(JP)**
Inventor: **SUGIYAMA, Makoto**
**6-23, Otowa Otsudecho Yamashina-ku**
**Kyoto-shi Kyoto 607(JP)**

(74) Representative: **Kügele, Bernhard**
**c/o NOVAPAT-CABINET CHEREAU 9, Rue du Valais**
**CH-1202 Genève(CH)**

(54) **FAT EMULSION.**

(57) A fat emulsion mainly comprising 0.001 to 10 % (w/v) of a mitomycin C derivative of general formula (I), 0.5 to 30 % (w/v) of a simple lipid, a phospholipid in an amount of 0.05 to 2 times by weight as large as the simple lipid, and an appropriate amount of water: (I) wherein X represents $-(CH_2)_n-O-$ or $-(CH_2)_n-NHCOO-$; n represents an integer of 0 to 4; and R represents a linear or branched, open-chain or cyclic, saturated or unsaturated hydrocarbon group containing 3 to 25 carbon atomes. This emulsion causes efficient migration of a prodrug of mitomycin C with a carcinostatic action in the form of an injection into the tissues, helps to maintain its blood level, and allows safe administration of an effective dose.

EP 0 510 197 A1

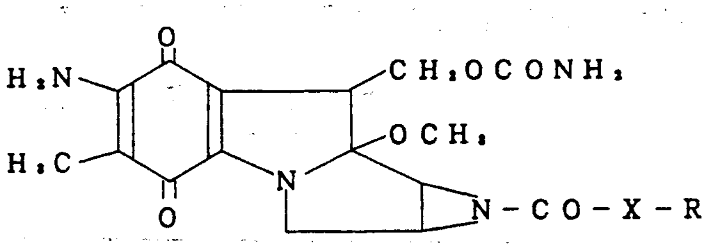

〔I〕

(Technical Field)

The present invention relates to pharmaceutical preparations of mitomycin C derivatives having antitumor activity. More particularly, it relates to antitumor preparations containing mitomycin C derivatives, simple lipid, phospholipid and water.

(Background Art)

Mitomycin C exhibits excellent antitumor action and has been widely used in clinical field. However, it exhibits many severe side effects and there are disadvantages that clinical use of the preparation of this substance is significantly restricted whereby sufficient drug therapy is not possible. Accordingly, there has been a demand for improvement of transfer of mitomycin C to the tumor parts and also for elimination of the side effects.

Up to this date, various derivatives of mitomycin C have been screened and many efforts have been done to solve the above-given disadvantages by pharmaceutical means represented by liposome concerning mitomycin C derivatives (for example, nonyloxycarbonylmitomycin C) expressed by the general formula (I) which is a prodrug for mitomycin C.

After administration, the liposome preparation of the above-given mitomycin C derivatives quickly releases the drug therefrom and is easily metabolized whereby satisfactory improvement in antitumor activity is not resulted and, consequently, more improvement has been demanded.

Further, various investigations on the device in pharmaceutical preparations for improving the transfer of the drug from blood or applied part to the lesion tissue. However, in a method wherein the drug is incorporated in liposome prepared from the phospholipid, there are several disadvantages. They are (1) there are many problems in the liposome wherein aqueous layer is covered by bilayers of lipid in terms of stability during storage; (2) the above-given mitomycin C derivatives are highly oil-soluble in oil and, when they are made into liposome preparations, they damage the liposome membrane structure due to their presence in the bi-molecular membranes of phospholipid and, accordingly, it is not possible to keep the drug in stable form.

This is probably due to the fact that, since the liposome in such a structure that it separates the aqueous layers both inside and outside by the bimolecular membrane of phospholipid, the liposome is not so stable against various forces. In addition, an increase in particle size due to coagulation has been known as another disadvantage on storage.

(Disclosure of the Invention)

The present inventors have conducted investigations for offering safe, effective and novel pharmaceutical preparations whereby the mitomycin C derivatives can be stably held and their transfer to the tumor part can be improved and have at last succeeded in completion the present invention.

Usually, the administered drug moves and is distributed in the body depending upon the nature inherent to the drug molecule. Upon arrival to the acting part, the effect is achieved. It is preferred that, at that time, the drug is concentrated only to the part where achievement of the effect is expected but, usually, the drug is distributed throughout the body whereby the drug transfer even to unnecessary part. Sometimes, it is a cause of side effects. Consequently, it is important and necessary to improve the behavior of the drug in vivo.

Under such circumstances, the present inventors have conducted various investigations on safe and more effective preparations of mitomycin C derivatives meeting with the following requirements. (1) the pharmacological action per se of the drug is not affected; (2) selective and effective transfer of the drug to the lesion tissue is possible; and (3) the level of the drug in blood can be maintained. As a result thereof, the present inventors have finally succeeded in accomplishing the present invention.

The characteristic feature of the present invention is that, in manufacturing the fat emulsion containing mitomycin C derivative as a main component, the composition ratio of the constituting ingredients - simple lipid, phospholipid and water - is specified.

The mitomycin C derivatives in accordance with the present invention may be expressed by the following general formula (I):

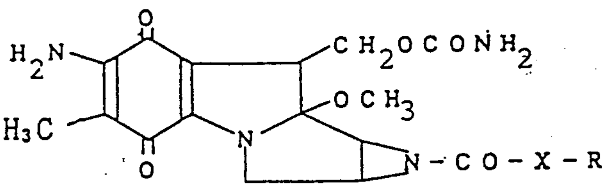

( I )

wherein X is $-(CH_2)_n-O-$ or $-(CH_2)_n-NHCOO-$ (n is an integer of 0-4) and R is $C_{3-25}$ straight, branched or cyclic, saturated or unsaturated hydrocarbon.

Examples of R are isobutyl, nonyl, cetyl, geranyl and cholesteryl though R is not limited thereto.

The compounds of the present invention expressed by the general formula (I) are novel,and have not been disclosed in the prior art literature and have been firstly found by the present inventors except alkyloxycarbonylmitomycin C (X is $-(CH_2)_n-O-$ and n is 0 in (I)) and the mitomycin C derivatives wherein R is cholesteryl (e.g. N-cholesteryloxycarbonylglycylmitomycin C and cholesteryloxyacetylmitomycin C, etc.).

The mitomycin C derivatives in accordance with the present invention are metabolized to mitomycin C in vivo in a rate which is different from that of known mitomycin C derivatives. Therefore, they are useful as prodrugs for mitomycin C.

The compounds expressed by the general formula (I) are particularly excellent in free mitomycin C production in human blood plasma and tumor cells. The mitomycin C derivatives per se do not exhibit activity but, upon being metabolized to mitomycin C (an active form) in vivo including tumor, the efffect is firstly resulted.

It is preferred that, in the present invention, the concentration of the mitomycin C derivative in the entire fat emulsion is not more than 10% (w/v).

In the present invention, the simple lipid contained in the entire fat emulsion is to be made 0.5-30% (w/v).

In the present invention, the amount of the phospholipid to the above simple lipid is to be 0.05 to 2 times as much by weight.

If the amount is less than the above, contamination of big particles cannot be avoided whereupon it is not possible to prepare stable fat emulsion containing the drug while, if the amount of the phospholipid is more than that, contamination of liposome particle cannot be avoided whereupon it is not possible to give homogeneous fat emulsion.

Water which is one of the components of the present invention may be in suitable amount.

It has been for the first time made clear that the composition in the above-given ratio affords stable emulsion of microparticles which is a pharmaceutical preparation exhibiting significant characteristics and is capable of utilizing as novel mitomycin C derivative preparation.

The fat emulsion of the present invention can hold the mitomycin C derivatives in the emulsion particles in stable form.

Mean particle size of the fat emulsion of the present invention is not larger than 0.5 micron. The fat emulsion of the present invention is stable containing no emulsion particles in the size of 1 micron or larger.

The emulsion particles of the fat emulsion of the present invention is capable of effective transfer of the drug to lesion part because of the following reasons. (1) The particles move to tumer cells and phagocytes such as macrophage accumulated at certain place by bio-protective reaction and inflammation due to tumer; (2) In tumer blood vessels, there is an increased permeability through blood vessels and, accordingly, they are easily and selectively oozed into tissue, etc.

In addition, with regard to the emulsion particles of not larger than about 100 nm, their nonspecific incorporation by reticuloendothelial system (RES) such as liver can be avoided whereupon the effect of holding the concentration of the drug in blood in more high level can be resulted. That will further results in transfer of the emulsion particles of the present invention to the tumor tissue in much more amounts.

As the emulsion particles move into the lesion, the drug entrapped in the emulsion particles also move thereinto. As such, the drug is easily and selectively transfers into the lesion whereupon the drug concentrations in the lesion become higher and the effect can be further increased.

In accordance with the present invention, the mitomycin C derivative is present in the oil droplets of the lipid and, therefore, it exists in an isolated state from the external circumstances whereby its enzymatic and nonenzymatic decomposition can be prevented and, even after administration, stability of the drug can be improved.

Consequently, mitomycin C of active form which is liberated from the mitomycin C derivatives of the

present invention acts in the lesion tissue in an sustained basis.

Examples of the simple lipid used in the fat emulsion of the present invention are neutral lipid such as refined soybean oil, cotton seed oil, rape seed oil, sesame oil, corn oil, peanut oil, sufflower oil, triolein, trilinolein, tripalmitin, tristearin, trimyristin and triarachidonin. Other examples are sterol derivatives such as cholesteryl oleate, cholesteryl linolate, cholesteryl myristate, cholesteryl palmitate, cholesteril arachidate and the like. This is because neutral lipids are relatively easily decomposed by various lipases existing in internal skin of blood vessel while the cholesterol derivatives are hardly decomposed those enzymes whereby they are most stable in vivo.

Examples of the phospholipid are those derived from egg yolk, soybean, cow, swine, etc. and those obtained by purely synthetic or semisynthetic route such as, for example, phosphatidyl choline, phosphatidyl ethanolamine, phosphatidyl serine, phosphatidyl inositol and phosphatidyl glycerol. More specific examples will be egg yolk phosphatidyl choline, soybean phosphatidyl choline, dipalmitoyl phosphatidyl choline, dimyristoyl phosphatidyl choline, distearoyl phosphatidyl choline, dioleoyl phosphatidyl choline and dipalmitoyl choline, distearoul phosphatidyl choline and dipalmitoyl phosphatidyl glycerol. Their hydrogenated products may be used as well. Particularly preferred representative example will be refined egg yolk lecithin. In order to give charge on the surface of the emulsion particles, lipid having charges such as, for example, stearylamine, dicetyl phosphate, phosphatidic acid and phosphatidyl glycerol may be used too.

In the manufacture of the fat emulsion of the present inventon and of the preparation using the same, various kinds of methods for the manufacture of emulsion used conventionally may be directly applied.

For example, the whole components including the drug are well atomized using Manton-Gaurin compressie spraying homogenizer, microfluidizer, ultrasonic homogenizer, etc. Such methods are most common. In that case, commonly-known auxiliary emulsifiers or stabilizers such as physiologically-acceptable sterols, fatty acids or derivatives thereof may be added thereto. Representative examples of them are cholesterol and oleic acid , etc.

Shape and particle size of the fat emulsion of the present invention can be easily confirmed by analyzing devices for particle size utilizing scattering and electron microscope and by filtering through membrane filter.

Besides the above-mentioned essential components of the present invention, the preparations of the fat emulsion of the present invention may contain additives and auxiliary substances which have been commonly used in injections. For example, antioxidants, antiseptics, stabilizers, isotonic agents, buffers, etc. may be added thereto. Minimum amount and optimum amount of such additives and auxiliary substances may vary depending upon the object.

The pharmaceutical preparations of the present invention manufactured as such may, for example, be sterilized (by means, for example, of filtration or high pressure steam) and sealed in ampoules. If necessary, they may be lyophilized. The lyophilized pharmaceutical preparations of the present invention may be revived by adding suitable solution.

Usually, the fat emulsion of the present invention is intravenously administered to human being or animals as a remedy for various malignant tumor or the like. In that case, particle size of the emulsion particles should be well controlled because, if there are particles of the size of 1 micron or larger, various toxities are known to happen.

If necessary, the fat emulsion of the present invention may be administered as injection into artery, muscle or skin. Further, the pharmaceutical preparations of the present invention may be in a form of giving via eye, nose, mouth, etc. or may be inhated or injected into bladder or may be in a form of suppositories or ointment. In those cases, pharmaceutically-acceptable base materials, fillers and other additives may be optional components therefor.

Dose of the pharmaceutical preparation comprising the fat emulsion of the present invention may vary depending upon the administering route, dosage form, symptoms of the disease, object, etc. but, usually, 1 to 1,000 ml per administration as an emulsion.

(Merit)

In accordance with the present invention, the clinical use value of mitomycin C derivatives can be greatly increased. The merit or effect of the present invention is that the above-given conventional problems are solved, antitumor effect is improved and the side effect or toxicity which is the biggest problem in clinical application can be greatly reduced whereby novel pharmaceutical preparation of mitomycin C with safety and much more effectiveness can be achieved. Such a merit or effect can firstly be achieved by the present invention.

The components used for the fat emulsion of the present invention are mostly composed of lipid which has been used as pharmacologically-acceptable substance in clinical field and, therefore, they can be used with great safety.

(Best Mode of Conducting the Invention)

The present invention will be further illustrated by way of the examples for manufacturing fat emulsion of the present invention which, however, are never intended to limit the scope of the present invention thereto.

Manufacturing Example 1.

Nonyloxycarbonylmitomycin C (3 mg), 0.5 g of pure soybean oil and 0.5 g of pure egg yolk lecithinwere dissolved in 100 ml of 1:1 (by volume) mixture of chloroform and methanol and the solvents were completely removed in vacuo using a rotary evaporator. To the residue was added 8 ml of 0.24M aqueous solution of glycerol and the mixture was stirred using a homogenizer to give a crudely emulsified mixture. To this was added aqueous solution of 0.24M glycerol to make the whole volume 10 ml. This was then emulsified, with ice cooling, using an ultrasonic homogenizer (Brandon, Model 185) for 60 minutes to give very fine fat emulsion containing mitomycin C derivative.

Manufacturing Example 2.

Nonyloxycarbonylmitomycin C (2 g), 50 g of pure soybean oil and 6 g of pure egg yolk lecithin were mixed by warming at about 60° C, then 500 ml of 0.24M aqueous solution of glycerol was added and the mixture was stirred with a homomixer to give a crudely emulsified mixture. This is subjected to a high pressure emulsification using a Manton-Gaurin homogenizer to give very fine fat emulsion containing mitomycin C derivative.

Manufacturing Example 3.

Nonyloxycarbonylmitomycin C (40 mg), 0.5 g of pure soybean oil and 0.5 g of pure egg yolk lecithin were dissolved in 100 ml of 1:1 (by volume) mixture of chloroforma and methanol and then the solvent was completely removed in vacuo using a rotary evaporator. To this was added 8 ml of isotonic phosphate buffer and the mixture was stirred using a homogenizer to give a preliminarily emulsified mixture. To this was added an isotonic phosphate buffer to make the volume 10 ml. This was emulsified, with ice cooling, using an ultrasonic homogenizer (Branson, Model 185) for 60 minutes to give fat emulsion containing very fine mitomycin C derivative.

Manufacturing Example 4.

N-(Cholesteryloxycarbonyl)-glycylmitomycin C (2 g), 20 g of pure soybean oil and 25 g of pure egg yolk lecithin were mixed with warming at about 60° C. To this was added 100 ml of 0.24M aqueous solution of glycerol and the mixture was stirred using a homomixer to give a preliminarily emulsified mixture. This was subjected to a high pressure emulsification using a microfluidizer to give very fine fat emulsion containing mitomycin C derivative.

Manufacturing Example 5.

Nonyloxycarbonylmitomycin C (1 mg), 0.5 g of cholesteryl oleate and 0.5 g of pure egg yolk lecithin were dissolved in 100 ml of 1:1 (by volume) mixture of chloroform and methanol and then the solvent was completely removed in vacuo using a rotary evaporator. To this was added 8 ml of 0.24M aqueous solution of glycerol and the mixture was stirred using a homogenizer to give a preliminarily emulsified mixture. To this was added 0.2M aqueous solution of glycerol to make the volume 10 ml followed by homogenizing for 60 minutes using an ultrasonic homogenizer (Brandon Model 185) to give very fine fat emulsion containing mitomycin C derivative.

Manufacturing Example 6.

Cholsteryloxyacetylmitomycin C (3 mg), 0.5 g of pure soybean oil, 0.4 g of pure egg yolk lecithin and 0.1 g of dimyristoylphosphatidyl glycerol were dissolved in 100 ml of a 1:1 (by volume) mixture of chloroform and methanol and then the solvent was comletely removed in vacuo using a rotary evaporator. To this was added 8 ml of 9% aqueous solution of lactose and the mixture was stirred using a homogenizer to give a preliminarily emulsified mixture. To this was added 9% aqueous solution of lactose to make the volume 10 ml followed by homogenizing for 60 minutes using an ultrasonic homogenizer (Branson Model 185) to give very fine fat emulsion containing mitomycin C derivative.

Manufacturing Example 7.

Nonyloxycarbonylmitomycin C (5 mg), 0.5 g of pure soybean oil, 0.4 g of hydrogenated egg yolk lecithin and 0.1 g of cholesterol were dissolved in a 1:1 (by volume) mixture of chloform and methano and then the solvent was completely removed in vacuo using a rotary evaporator. To this was added 8 ml of 9% aqueous solution of lactose followed by stirring with a homogenizer to give a preliminarily emulsified mixture. To this was added 9% aqueous solution of lactose to make the volume 10 ml followed by emulsifyling by an ultrasonic homogenizer (Branson Model 185) for 60 minutes to give very fine fat emulsion containing mitomycin C derivative.

Manufacturing Example 8.

N-(Nonyloxycarbonyl)glycylmitomycin C was obtained by the synthetic route as given below.

A solution of 824 mg of nonyl chlorocarbonate in 20 ml of dioxane was dropped, at 0° C, into a solution of 300 mg of glycine and 808 mg of triethylamine in 3 ml of water. The mixture was stirred for 3 hours, the reaction solution was concentrated in vacuo, 10 ml of 1N hydrochloric acid was added to the residue and the resulting precipitate was extracted with 20 ml of chloroform. The organic layer was washed with 10 ml of water, dried with magnesium sulfate and concentrated in vacuo to give 570 mg of N-(nonyloxycarbonyl)-glycine, white crystals. The crystals were dissolved in a mixture of 10 ml of dioxane and 2 ml of chloroform and, at 0° C, 270 mg of N-hydroxysuccinimide and 470 mg of dicyclohexylcarbodiimide were added thereto. The mixture was allowed to stand at 4° C for 12 hours, the separated precipitate was removed by filtration and the filtrate was concentrated in vacuo to give colorless oily residue in an amount of 772 mg.

The above oily residue (150 mg) and 36 mg of pyridine were added, at room temperature, to a solution of 150 mg of mitomycin C in 5 ml of N,N-dimethylformamide and the mixture was stirred for 4 hours. The reaction solution was concentrated in vacuo and the residue was dissolved in 20 ml of chloroform followed by washing with 15 ml of water. The organic layer was dried with magnesium sulfate and concentrated in vacuo to give reddish purple residue. The residue was subjected to a silica gel column chromatography and eluted with a mixture of chlorofrom and methanol. The reddish purple fraction was concentrated in vacuo to give 225 mg (yield: 89%) of N-(nonyloxycarbonyl)-glycylmitomycin C, reddish purple crystals, m.p. being not lower than 250° C (decomposition).

Its FAB-MS spectrum showed m/2: 563 (M + 2) and $^1$H-NMR spectra showed the following peaks whereby the structure was confirmed.

δ: 5.36-5.08 (3H, m, $-COCH_2NHCOO-$, $NH_2$)
4.43 (1H, d, J = 13.5 Hz, 3-H)
4.16-3.89 (4H, m, $-COCH_2NH-$, $-COOCH_2-$)
3.71 (1H, dd, J = 10.8Hz, 10-H)
3.59 (1H, d, J = 4.6 Hz, 10-H0
3.57 (1H, dd, J = 1.6, 13.5Hz, 3'-H)
3.48 (1H, dd, J = 1.6, 4.6Hz, 2-H)
3.18 (3H, s, $OCH_3$)
1.78 (4H, s, $-C=C-CH_3$)
1.66-1.48 (2H, m, $-COOCH_2CH_2-$)
1.42-1.16 (12H, m, $-CH_2(CH_2)_6CH_2$)
0.83 (3H, t, J = 7Hz, $-(CH_2)_8CH_3$

N-(Nonyloxycarbonyl)glycylmitomycin C prepared as such (3 mg), 0.5 g of pure soybean oil and 0.5 g of pure egg yolk letichin were dissolved in 100 ml of 1:1 (by volume) mixture of chloroform and methanol and then the solvent was completely removed in vacuo using a rotary evaporator. To this was added 8 ml of 0.24M aqueous solution of glycerol followed by stirring by a homogenizer to give a preliminarily emulsified mixture. To this was added 0.24M aqueous solution of glycerol to make the volume 10 ml followed, under ice cooling, by emulsifying for 60 minutes using an ultrasonic homogenizer (Branson Model

185) to give very fine fat emulsion containing mitomycin C derivative.

Manufacturing Example 9.

N-(Isobutyloxycarbonyl)glycylmitomycin C was obtained by a method as given below.

A solution of 544 mg of isobutyl chlorocarbonate in 20 ml of dioxane was dropped, at 0°C, into a solution of 300 mg of glycine and 808 mg of triethylamine in 3 ml of water. The mixture was stirred for 3 hours, the resulting reaction solution was concentrated in vacuo, 10 ml of 1N hydrochloric acid was added to the residue and the precipitate separated out therefrom was extracted with 20 ml of chloroform. The organic layer was washed with 10 ml of water, dried with magnesium sulfate and concentrated in vacuo to give 409 mg of N-(isobutyloxycarbonyl)glycine, white crystals.

The crystals were dissolved in a mixture of 10 ml of dioxane and 2 ml of chlroform at, at 0°C, 270 mg of N-hydroxysuccinimide and 470 mg of dicyclohexylcarbodiimide were added thereto. The mixture was allowed to stand at 4°C, the separated precipitate was filtered off and the filtrate was concentrated in vacuo to give 772 mg of colorless oily residue.

The above oily residue (150 mg) and 36 mg of pyridine were added, at room temperature, to a solution of 150 mg of mitomycin in 5 ml of N,N-dimethylformamide and stirred for 4 hours. The reaction solution was concentrated in vacuo and the residue was dissolved in 20 ml of chloroform and washed with 15 ml of water. The organic layer was dried with magnesium sulfate and concentrated in vacuo to give reddish purple residue. The residue was subjected to silica gel column chromatography and eluted with a mixture of chloroform/methanol. The resulting reddish purple fraction was concentrated in vacuo to give 161 mg of N-(isobutyloxycarbonyl)glycylmitomycin C, reddish purple crystals, m.p. higher than 250°C (decomposition). The yield was 73%.

The resulting N-(isobutyloxycarbonyl)glycylmitomycin C (3 mg), 0.5 g of pure soybean oil and 0.5 g of pure egg yolk lecithin were dissolved in 100 ml of a 1:1 (by volume) mixture of chloroform and methanol and the solvent was completely removed in vacuo using a rotary evaporator. To this was added 8 ml of 0.24M aqueous solution of glycerol and the mixture was stirred with a homogenizer to give a preliminarily emulsified mixture. To this was added 0.24M aqueous solution of glycerol to make the volume 10 ml and, with ice cooling, the mixture was emulsified for 60 minutes using an ultrasonic homogenizer (Branson Model 185) to give very fine fat emulsion containing mitomycin C derivative.

Manufacturing Example 10.

Cetyloxyacetylmitomycin C was obtained in accordance with the following synthetic route.

Cetyl alcohol (1.2 g) and 10 ml of ethylene glycol were added to 100 ml of benzene, then 100 mg of p-toluenesulfonic acid monohydrate was added thereto and the mixture was heated to reflux for 8 hours. The reaction mixture was concentrated in vacuo, the residue was extracted with ether, the extract was washed with saturated aqueous solution of sodium bicarbonate, dried with magnesium sulfate and concentrated in vacuo to give a residue. The residue was dissolved in ether, the solution was dropped into a solution of 0.5 g of lithium aluminum hydride an 2 g of aluminum chloride in 50 ml of ether and the mixture was stirred for 4 hours. After adding certain amount of diluted sulfuric acrid thereto, the insoluble matters were filtered off and the ether layer was washed with water and 5% sodium bicarbonate, dried with magnesium sulfate and concentrated in vacuo to give a residue. This oily residue was oxidized with Jones reagent to give cetyloxyacetic acid.

The resulting cetyloxyacetic acid was bonded with mitomycin C using N-hydroxysuccinimide by the same manner as in the method of Manufacturing Example 8 to give cetyloxyacetylmitomycin C, m.p. 110-120°C (decomposition).

The resulting cetyloxyacetylmitomycin C (3 mg), 0.5 g of pure soybean oil and 0.5 g of pure egg yolk lecithin were dissolved in 100 ml of a 1:1 (by volume) mixture of chloroform and methanol and the solvent was completely evaporated in vacuo using a rotary evaporator. To the residue was added 8 ml of 0.24M aqueous solution of glycerol and the mixture was homogenized with stirring to give a preliminarily emulsified mixture. To this was added 0.24M aqueous solution of glycerol to make the volume 10 ml and the mixture was emulsified, with ice cooling for 60 minutes using an ultrasonic homogenizer (Branson Moedl 185) to give very fine fat emulsion containing mitomycin C derivative.

Manufacturing Example 11.

N-(Geranyloxycarbonyl)glycylmitomycin C (m.p. being not lower than 250°C [decomposition]) was

synthesized by the same operation as in Manufacturing Example 8 using the same mole of geranyl chlorocarbonate instead of nonyl chlorocarbonate used in Manufacturing Example 8. This mitomycin C derivative (40 mg), 0.5 g of pure soybean oil and 0.5 g of pure egg yolk lecithin were dissolved in 100 ml of a 1:1 (by volume) mixture of chloroform and methanol and the solvent was completely remoed in vacuo using a rotary evaporator. To the residue was added 8 ml of isotonic phosphate buffer and the mixture was stirred using a homogenizer to give a preliminarily emulsified mixture. To this was added isotonic phosphate buffer to make the volume 10 ml. The mixture was emulsified, with ice cooling for 60 minutes using an ultrasonic homogenizer (Branson Model 185) to give very fine fat emulsion containing mitomycin C derivative.

Manufacturing Example 12.

To the fat emulsions obtained in Manufacturing Examples 1, 5, 6 and 8 were added 0.5 g each of albumin and the mixtures were lyophilized to give dry preparations.

Results of the test on evaluation of characteristics of the fat emulsions of the present invention will be given as hereunder.

In the Test Examples 1, 2 and 3, the fat emulsion of the present invention obtained in Manufacturing Example 3 was used as a test sample while, in Test Example 4, the fat emulsion of the present invention obtained in Manufacturing Example 4 was used as a test sample.

For comparison, commerically-available mitomycin C injection solution (Mitomycin C Kyowa S [trademark]) manufactured by Kyowa Hakko) was used.

Test Example 1. Test on Evaluation of Toxicity.

Male mice of ddY strain (body weight: about 30 g) were used as experimental animals and the test sample or the control sample was intravenously injected from tail vein. The dose calculated as free mitomycin C was 5 mg/kg. Injection was conducted every another day and carried out for three times. The average body weight of the mice 4 days after the final injection is given in Table 1.

Mice which were injected with the test samples showed a normal body weight increase the same as that which was not injected. No toxicity was noticed. However, in the mice injected with the control sample, much body weight decrease was noticed and, in addition, severe damage of digestive organs was observed.

Table 1

| Evaluation of Toxicity by Body Weight Changes | |
|---|---|
| | Av.Body Wt.of Mice |
| Not injected | 34.0 g |
| Injected with test sample | 33.6 g |
| Injected with control sample | 22.0 g |
| (Averages of ten mice each) | |

The fat emulsion of the present invention exhibited significantly lower toxicity than the conventionally-known mitomycin C preparations and it is clear that far more safe therapy by drugs can be achieved.

Test Example 2. Evaluation of Antitumor Activity.

Male mice (body weight: ca. 20 g) of CDF1 strain were used as experimental animals. After intraperitoneal administration (inoculation) of P 388 tumor cells, the test sample or the control sample was intravenously injected from tail vein twice - i.e. 2 days and 4 days thereafter - for therapy. Dose calculated as free mitomycin C was 5 mg/kg for each injection. With regard to the control group, physiological saline solution was intravenously injection by the same manner. The life-prolonging effect calculated by a comparison with the control group (i.e. ILS%) is given in Table 2.

Table 2

| Antitumor Effects | |
|---|---|
| | Increased Life Span (ILS%) |
| Test Sample | 75.8 |
| Control Sample | 16.5 |

It is noticeable that the antitumore effect (life-prolonging rate) of the test sample was far more better than that of the control sample.

It is therefore clear that the fat emulsion of the present invention achieved more effective and safe drug therapy by the fat emulsion of the present invention because of so significantly improved antitumor effect (life-prolonging rate) than the conventionally-known mitomycin C preparation.

Test Example 3. Changes in Concentrations in Blood.

Male mice (body weight: ca. 25 g) of CDF1 strain were used as experimental animals and the test sample and the control sample were intravenously injected from tail vein. The dose calculated as free mitomycin C was 5 mg/kg. After 1, 30 and 60 minutes, small amounts of blood were collected. The mitomycin C concentrations in blood were measured by means of high performance liquid chromatography and the result is given in Table 3.

Table 3

| Concentrations in Blood ( µg/ml) | | | |
|---|---|---|---|
| | After 1 Min | After 30 Min | After 60 Min |
| Test Sample | 0.1 | 0.6 | 0.5 |
| Control Sample | 4.5 | 0.4 | 0.1 |
| (Averages of three mice each) | | | |

It is observed that the changes in mitomycin C concentrations in blood when the test sample was injected were more highly sustained than those of the control sample.

It is therefore clear that the fat emulsion of the present invention achieved the sustained mitomycin C concentration in blood as compared with the conventionally-known mitomycin C preparation.

The very high mitomycin C concentration immediately after injectino of the control sample was not observed in the case of the test sample. Thus, the present invention is favorable in terms of toxicity as well.

Test Example 4. Movement of the Drug to Tumor Part.

Male mice of ddY strain (body weight: ca: 25 g) were used as experimental animals. S-180 tumor cells were administered hypodermically and, 10 days after that, the test sample or the control sample was intravenously injected from tail vein. The dose calculated as free mitomycin C was 5 mg/kg. After 30 minutes, solid tumor was excised from the mice, bomogenized and the total mitomycin C concentrations (calculated as mitomycin C) in tumor were measured by means of high performance liquid chromatography. The result is given in Table 4.

Table 4

| Total Mitomycin C Concentrations in Tumor | |
|---|---|
| | Total Mitomycin C Concn |
| Test Sample | 0.84 ± 0.22 |
| Control Sample | 0.15 ± 0.08 |
| (average ± standard deviation) Numbers of mice: three | |

It is observed that the mitomycin C concentrations in tumor when the test sample was given were higher than those of the control sample.

It is therefore clear that the fat emulsion of the present invention exhibits significant accumulation to tumor part and achieved the more effective and safe drug therapy as compared with the conventionally-known mitomycin C preparation.

Test Example 5. Production of mitomycin C from mitomycin C derivative.

Production of mitomycin C from nonyloxycarbonylmitomycin C (Derivative 1), N-(nonyloxycarbonyl)-glycylmitomycin C (Derivative 2) and N-(cholesteryloxycarbonyl)-glycylmitomycin C (Derivative 3) is given in Table 5.

S-180 solid tumor promulgated under the skin of mice of ddY-strain and human-derived solid tumor cells (MX-1) promulgated under skin of nude mice were excised and each was made into 10% homogenate using physiological saline solution which was buffered with isotonic phosphate. To this was added each of the above mitomycin C derivatives to make the final concentration 10 µM and, after incubating at 37° C for 60 minutes, the resulting free mitomycin C was measured by means of high performance liquid chromatography.

It is clear that all of the mitomycin C derivatives produced free mitomycin which is an activated form in tumor. There was an exactly quantitative relationship between the decomposition of the added mitomycin C derivative and the production of the free mitomycin C.

It is now confirmed that those derivatives exhibit the property as prodrugs for mitomycin C.

Production rate of free mitomycin C varied depending upon the derivatives and N-(nonyloxycarbonyl)-glycylmitomycin C (Derivative 2) was quicker in the liberation of mitomycin C as compared with Derivatives 1 and 3. The same result was obtained in the case of human-derived tumor cells.

Table 5

| Production of Mitomycin C (%) | | |
|---|---|---|
| | S-180 | MX-1 |
| Derivative 1 | 3.0 | 5.7 |
| Derivative 2 | 16.0 | 11.4 |
| Derivative 3 | 10.7 | 8.3 |

Test Example 6. Measurement of Particle Size.

Fat emulsions of the Manufacturing Examples 2 and 3 were evaluated by measuring their particle sizes using dynamic light scattering particle size measuring machine wherein laser was used.

The result was that the average particle size of the Manufacturing Example 2 was about 150-250 nm and contained no particles of not smaller than 1 µ and that the size of Manufacturing Example 3 was about 20-100 nm and contained no particle of not smaller than 1 µ.

The fat emulsion of the present invention contains very small and uniform particles and contains no particles of 1 µ or larger size which result in a problem of toxicity. Therefore, it is obvious that effective and safe drug therapy can be now achieved.

(Utilizability in Industry)

As fully illustrated hereinabove, mitomycin C derivatives which are prodrugs of mitomycin C can be safely administered in an effective dose in accordance with the present invention. Thus, the present invention is useful in pharmaceutical industry.

## Claims

**1.** Fat emulsion (or lyophilized preparation thereof) containing the following components:
(a) 0.001-10% (w/v) of mitomycin C derivative expressed by the following general formula (I)

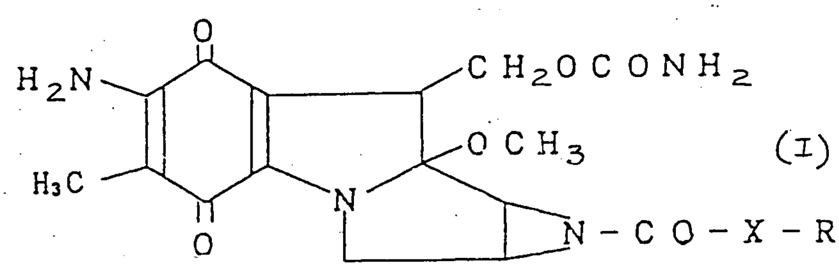

(I)

(wherein X is $-(CH_2)_n-O-$ or $-(CH_2)_n-NHCOO-$ [n is an integer of 0-4] and R is $C_{3-25}$ straight or branched, open-chain or cyclic and saturated or unsaturated hydrocarbon);
(b) 0.5-30% (w/v) of simple lipid;
(c) phospholipid in an amount of 0.05-2 times by weight as much as the simple lipid; and
(d) an appropriate amount of water

# INTERNATIONAL SEARCH REPORT

International Application No PCT/JP91/00006

**I. CLASSIFICATION OF SUBJECT MATTER** (if several classification symbols apply, indicate all) [6]

According to International Patent Classification (IPC) or to both National Classification and IPC

Int. $Cl^5$ A61K31/40, A61K9/107, A61K9/127, A61K9/14

**II. FIELDS SEARCHED**

Minimum Documentation Searched [7]

| Classification System | Classification Symbols |
|---|---|
| IPC | A61K31/40, A61K9/107, A61K9/127, A61K9/14 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | Chemical & Pharmaceutical Bulletin, Vol. 33, No. 7, (1985), H. Sasaki et al. Blood Dispositions of Mitomycin C and a Lipophilic Prodrug after Intravenous Administration in Liposomes and O/W Emulsion , p. 2968 - 2973 | 1 |
| A | JP, A, 60-115517 (Bayer A.G.), June 22, 1985 (22. 06. 85) & US, A, 4711902 & EP, A, 143305 | 1 |
| P | Journal of Organic Chemistry, Vol. 55, No. 9, (1990), P.D. Senter et al. Development of a drug-release strategy based on the reductive fragmentation of benzyl carbamate disulfides , p. 2975 - 2978 | 1 |
| P | JP, A, 2-167217 (Shiseido Co., Ltd.), June 27, 1990 (27. 06. 90), Upper left column, page 3, | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| March 27, 1991 (27. 03. 91) | April 8, 1991 (08. 04. 91) |
| **International Searching Authority** | **Signature of Authorized Officer** |
| Japanese Patent Office | |

International Application No. PCT/JP91/00006

**FURTHER INFORMATION CONTINUED FROM THE SECOND SHEET**

upper column, page 8
(Family: none)

**V. ☐ OBSERVATIONS WHERE CERTAIN CLAIMS WERE FOUND UNSEARCHABLE [1]**

This international search report has not been established in respect of certain claims under Article 17(2) (a) for the following reasons:

1. ☐ Claim numbers , because they relate to subject matter not required to be searched by this Authority, namely:

2. ☐ Claim numbers , because they relate to parts of the international application that do not comply with the prescribed requirements to such an extent that no meaningful international search can be carried out, specifically:

3. ☐ Claim numbers , because they are dependent claims and are not drafted in accordance with the second and third sentences of PCT Rule 6.4(a).

**VI. ☐ OBSERVATIONS WHERE UNITY OF INVENTION IS LACKING [2]**

This International Searching Authority found multiple inventions in this international application as follows:

1. ☐ As all required additional search fees were timely paid by the applicant, this international search report covers all searchable claims of the international application.
2. ☐ As only some of the required additional search fees were timely paid by the applicant, this international search report covers only those claims of the international application for which fees were paid, specifically claims:
3. ☐ No required additional search fees were timely paid by the applicant. Consequently, this international search report is restricted to the invention first mentioned in the claims; it is covered by claim numbers:
4. ☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.